# EUROPEAN PATENT APPLICATION

(11) **EP 3 795 176 A1**
(43) Date of publication of application: **24.03.2021**
(21) Application number: 19804443.0
(22) Date of filing: 14.05.2019
(51) Int. Cl.: A61K 39/395, A61P 9/06, C07K 16/22

(54) **PHARMACEUTICAL COMPOSITION FOR SUPPRESSING ATRIAL FIBRILLATION HAVING AS ACTIVE INGREDIENT ANTI-HUMAN NGF ANTIBODY OR ANTIGEN-BINDING FRAGMENT THEREOF**

(30) Priority: 15.05.2018 JP 2018093616
(71) Applicant: Astellas Pharma Inc., Chuo-ku Tokyo 103-8411 (JP)
(72) Inventor: SHAKUSHIRO, Kohsuke, Tokyo 103-8411 (JP); AOKI, Toshiaki, Tokyo 103-8411 (JP)
(74) Representative: Gille Hrabal Partnerschaftsgesellschaft mbB Patentanwälte
(86) International application number: PCT/JP2019/019045
(87) International publication number: WO 2019/221097

(57) **Abstract**

A novel pharmaceutical composition for suppressing atrial fibrillation that has an action mechanism that does not affect the normal heart function is provided. A pharmaceutical composition including an anti-human NGF antibody or antigen binding fragment thereof as an active ingredient is provided.

SEQUENCE LISTING

<110>  Astellas Pharma Inc.

<120>  Pharmaceutical composition for suppression of the atrial fibrillation
using an anti-human NGF antibody or an antigen-binding fragment as an active
component

<130>  A19004A00

<150>  JP 2018-093616
<151>  2018-05-15

<160>  4

<170>  PatentIn version 3.5


<210>  1
<211>  675
<212>  DNA
<213>  Artificial Sequence

<220>
<223>  DNA encoding h1f.6 heavy chain fragment

<400>  1
caggtgcagc tacagcagtg gggcgcagga ctgttgaagc cttcggagac cctgtccctc      60

acctgcgctg tctatggtgg gtccttcagt ggttactact ggagctgggt ccgccagccc     120

ccagggaagg ggctggagtg gattggggaa atcgaccata gtggaagcac caacaacaac     180

ccgtccctca agagtcgagt caccatatca gtaggcacgt ccaagaacca gttctccctg     240

aagctgagct ctgtgaccgc cgcggacacg gctgtgtatt actgttcgag agatgggggc     300

cccgaatcgg ggatgggggc ttttgatatc tggggccaag ggacaatggt caccgtctcc     360

tcagcctcca ccaagggccc atcggtcttc cccctggcac cctcctccaa gagcacctct     420

gggggcacag cggccctggg ctgcctggtc aaggactact tccccgaacc ggtgacggtg     480

tcgtggaact caggcgccct gaccagcggc gtgcacacct tcccggctgt cctacagtcc     540

tcaggactct actcccttag tagcgtggtg accgtgccct ccagcagctt gggcacccag     600

acctacatct gcaacgtgaa tcacaagccc agcaacacca aggtggacaa gaaagttgag     660

cccaaatctt gtgac                                                      675


<210>  2
<211>  657
<212>  DNA
<213>  Artificial Sequence

<220>
<223>  DNA encoding h1f.6 light chain

<400>  2
gatattgtga tgactcagtc tccactctcc ctgcccgtca cccctggaga gccggcctcc      60

atctcctgca ggtctagtca gagcctcctg catagtaatg gattcaacta tttgggttgg 120

tacctgcaga agccagggca gtctccacag ctcctgatct atttgggttc taatcgggcc 180

tccggggtcc ctgacaggtt cagtggcagt ggatcaggca cagattttac tctgaaaatc 240

agcagagtgg aggctgagga tgttggggtt tattactgca tgcaagctct acaaactccg 300

tacacttttg gccaggggac caagctggag atcaaacgga ctgtggctgc accatctgtc 360

ttcatcttcc cgccatctga tgagcagttg aaatctggaa ctgcctctgt tgtgtgcctg 420

ctgaataact tctatcccag agaggccaaa gtacagtgga aggtggataa cgccctccaa 480

tcgggtaact cccaggagag tgtcacagag caggacagca aggacagcac ctacagcctc 540

agcagcaccc tgacgctgag caaagcagac tacgagaaac acaaagtcta cgcctgcgaa 600

gtcacccatc agggcctgag ctcgcccgtc acaaagagct tcaacagggg agagtgt 657

<210> 3
<211> 225
<212> PRT
<213> Artificial Sequence

<220>
<223> h1f.6 heavy chain fragment

<400> 3

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Val Arg Gln Pro Pro Gly Lys Gly Leu Glu Trp Ile
        35                  40                  45

Gly Glu Ile Asp His Ser Gly Ser Thr Asn Asn Asn Pro Ser Leu Lys
    50                  55                  60

Ser Arg Val Thr Ile Ser Val Gly Thr Ser Lys Asn Gln Phe Ser Leu
65                  70                  75                  80

Lys Leu Ser Ser Val Thr Ala Ala Asp Thr Ala Val Tyr Tyr Cys Ser
                85                  90                  95

Arg Asp Gly Gly Pro Glu Ser Gly Met Gly Ala Phe Asp Ile Trp Gly
            100                 105                 110

Gln Gly Thr Met Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp
225

<210>  4
<211>  219
<212>  PRT
<213>  Artificial Sequence

<220>
<223>  h1f.6 light chain

<400>  4

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Phe Asn Tyr Leu Gly Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu Gln Thr Pro Tyr Thr Phe Gly Gln Gly Thr Lys Leu Glu Ile Lys

            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

## Description

### TECHNICAL FIELD

The present invention relates to a novel pharmaceutical use of an anti-human NGF antibody or an antigen binding fragment thereof as a composition for suppressing atrial fibrillation.

### BACKGROUND ART

Arrhythmia is a pathological condition in which an anomaly develops in the cardiac rhythm. Arrhythmia is broadly classified into bradyarrhythmia in which the main observation is a condition of a slow pulse, and tachyarrhythmia in which the main observation is a condition of a fast pulse. Tachyarrhythmia may be classified according to the site where the anomaly develops into supraventricular arrhythmia, such as atrial fibrillation, etc., and ventricular arrhythmia, such as ventricular fibrillation, etc. (NPL1).

A type of supraventricular arrhythmia, namely atrial fibrillation, occurs as a result of a disorder in electric signals flowing in the atrium. When atrial fibrillation occurs, the decline in cardiac function may lead to an occurrence of cardiac failure. Also, as a result of blood congestion in the atrium, thrombus may develop and be carried to the cerebral artery where it forms an embolus. Thereby causing cerebral infarction and posing a clinical problem (NPL2).

Supraventricular arrhythmia, such as atrial fibrillation, also occurs after surgeries. As an example, the occurrence rate after cardiac surgeries is said to be 10 to 30%. The occurrence of postoperative atrial fibrillation is known to pose medical problems such as an increase in mortality rate or cerebral infarction occurrence, a prolonged length of stay, or an increase in medical costs (NPL3).

Various antiarrhythmic agents are used for the treatment and prophylaxis of atrial fibrillation. Because these pharmaceutical agents show beneficial effect by acting on ion channels or neurotransmitter receptors existing on the cardiac muscle cells, they affect not only the stimulus transmission or the contraction function of the abnormal section of the cardiac muscle, which is the origin where arrhythmia occurs, but also the normal sections of the cardiac muscle (NPL4). Hence, when using these pharmaceutical agents, it is necessary to be cautious of the development of side effects, such as arrhythmogenic effects. For example, the antiarrhythmic agent having an antagonistic action against potassium channel, such as amiodarone, is known to hold a risk of inducing lethal ventricular tachycardia (torsades de pointes) (NPL5). There is thus a desire for a novel pharmaceutical agent that does not affect normal cardiac functions.

It is well known that an anomaly in the autonomic nervous system plays an important part in the pathological condition of arrhythmia. The autonomic nerve may be broadly classified into a sympathetic nerve and a parasympathetic nerve. The ways in which the sympathetic nervous system and/or parasympathetic nervous system are involved in the occurrence of arrhythmia are known to vary widely by the type of arrhythmia. As an example, it is reported that whereas a simultaneous stimulation of both the sympathetic nervous system and parasympathetic nervous system causes atrial fibrillation, an enhanced stimulation of the sympathetic nervous system causes either ventricular fibrillation or ventricular tachycardia (NPL6). In other words, the mechanism of the occurrence of arrhythmia differs between atrial fibrillation, which is a supraventricular arrhythmia, and ventricular fibrillation or ventricular tachycardia, which are ventricular arrhythmia. It is further noted that a type of antiarrhythmic agent, lidocaine, is effective for arrhythmia of the ventricle, but not effective for arrhythmia of the atrium (NPL7), and each pharmaceutical agent exerts different fibrillation suppression effects against ventricular fibrillation and atrial fibrillation.

A damage in the cardiac muscle due to diseases such as a development disorder or myocardial infarction causes anomaly (unbalanced patterning or hyperinnervation) in the sympathetic nerve control of the damaged section of the cardiac muscle, and this anomaly is considered to be involved in the occurrence of arrhythmia. The cause of an anomaly in the innervation of the damaged section of the cardiac muscle is known to be a change in the expression of Semaphorin 3A, which is a neuronal axon guidance molecule, or the nerve growth factor (NGF) (NPL6). NGF is one of the neurotrophic factors, and plays an important part in the differentiation and growth of the nerve cell. In addition, NGF is reported to alter repeated firing of the sympathetic nerve or cause hyperinnervation, thereby involving itself in the induction of arrhythmia (NPL8 and 9).

In a study that induces ventricular fibrillation by applying electric stimulus to the left ventricle of a rat, it has been reported that the threshold of the currency required to induce ventricular fibrillation is increased by administering GK-1 peptide having an antagonist activity against a NGF receptor, tropomyosin receptor kinase A (TrkA) (NPL10). Meanwhile, it is considered as mentioned above that the supraventricular arrhythmia and ventricular arrhythmia differ in terms of their mechanism of occurrence and effective pharmaceutical agents, and the suppression of atrial fibrillation through inhibition of the NGF signal has not been reported so far.

Antibodies neutralizing the effect of human NGF reported thus far are fulranumab (PTL 1), MEDI-578 (PTL 2), REGN475 (PTL 3), 1-15 (N52D-A)-Fab'-PEG (PTL 4), and h1f.6 (PTL 5) as fully human anti-human NGF antibodies, and tanezumab (PTL 6) and PG110 (PTL 7) as humanized anti-human NGF antibodies.

### CITATION LIST

### PATENT LITERATURE

PTL 1: WO 2005/019266
PTL 2: WO 2006/077441
PTL 3: WO 2009/023540
PTL 4: WO 2013/022083
PTL 5: WO 2016/190263
PTL 6: WO 2004/058184
PTL 7: WO 2010/128398

### NON PATENT LITERATURE

NPL 1: Hiroshi KASANUKI, Revised edition, Visual Cardiovascular Disease Series 1 Arrhythmia, Medical View, 2000, pp 7-14
NPL 2: Koichi HAYAKAWA, et al., Atrial fibrillation/Flutter/Tachycardia, Igakushoin, 1999, pp 60-65 and 275-279
NPL3: Ann Thorac Surg, 2014, Vol.98, pp 527-533
NPL 4: Am Heart J, 2000, Vol. 140, pp 12-20
NPL 5: Europace, 2006, Vol.8, pp 1051-1053
NPL 6: Auton Neurosci, 2017, Vol.205, pp 1-11
NPL 7: J Mol Cell Cardiol, 1995, Vol.27, pp 831-846
NPL 8: J Neurophysiol, 2006, Vol.96, pp 946-958
NPL 9: Circ Res, 2015, Vol.116, pp 2005-2019
NPL 10: Human Physiol, 2012, Vol.38, pp 428-432

### SUMMARY OF INVENTION

### TECHNICAL PROBLEM

The problem to be solved by the present invention is to provide a novel pharmaceutical composition for suppressing atrial fibrillation having an action mechanism that does not affect the normal heart function.

### SOLUTION TO PROBLEM

The present inventors conducted extensive studies to solve the aforementioned problem, and consequently found that the a pharmaceutical composition comprising an anti-human NGF antibody or an antigen binding fragment thereof as the active ingredient is useful in suppressing atrial fibrillation, and completed this invention.

In other words, the present invention is a pharmaceutical composition for suppressing atrial fibrillation comprising an anti-human NGF antibody or an antigen binding fragment thereof, and a pharmaceutically acceptable carrier. An aspect of the present invention may take the following form.
[1] A pharmaceutical composition for suppressing atrial fibrillation comprising an anti-human NGF antibody or an antigen binding fragment thereof, and a pharmaceutically acceptable carrier.
[2] The pharmaceutical composition according to [1], wherein the antigen binding fragment of the anti-human NGF antibody is an anti-human NGF antibody Fab fragment.
[3] The pharmaceutical composition according to [2], wherein the anti-human NGF antibody Fab fragment is selected from a group consisting of (a) and/or (b) below:
   (a) an anti-human NGF antibody Fab fragment comprising a heavy-chain fragment consisting of an amino acid sequence shown by SEQ ID NO: 3 and a light chain consisting of an amino acid sequence shown by SEQ ID NO: 4;
   (b) an anti-human NGF antibody Fab fragment formed by a posttranslational modification of the anti-human NGF antibody Fab fragment of (a).
[4] The pharmaceutical composition according to [3] comprising an anti-human NGF antibody Fab fragment comprising a heavy-chain fragment consisting of an amino acid sequence shown by SEQ ID NO: 3 and a light chain consisting of an amino acid sequence shown by SEQ ID NO: 4.
[5] The pharmaceutical composition according to [3] comprising an anti-human NGF antibody Fab fragment formed by a posttranslational modification of an anti-human NGF antibody Fab fragment comprising a heavy-chain fragment consisting of an amino acid sequence shown by SEQ ID NO: 3 and a light chain consisting of an amino acid sequence shown by SEQ ID NO: 4.
[6] The pharmaceutical composition according to [5] comprising an anti-human NGF antibody Fab fragment comprising a heavy-chain fragment consisting of an amino acid sequence shown by SEQ ID NO: 3, in which glutamine at amino acid position 1 of SEQ ID NO: 3 is modified to pyroglutamic acid, and a light chain consisting of an amino acid sequence shown by SEQ ID NO: 4.
[7] The pharmaceutical composition according to [3] comprising an anti-human NGF antibody Fab fragment comprising a heavy-chain fragment consisting of an amino acid sequence shown by SEQ ID NO: 3 and a light chain consisting of an amino acid sequence shown by SEQ ID NO: 4, and an anti-human NGF antibody Fab fragment comprising a heavy-chain fragment consisting of an amino acid sequence shown by SEQ ID NO: 3, in which glutamine at amino acid position 1 of SEQ ID NO: 3 is modified to pyroglutamic acid, and a light chain consisting of an amino acid sequence shown by . SEQ ID NO: 4.
[8] The pharmaceutical composition according to any one of [1] to [7], wherein the atrial fibrillation is postoperative atrial fibrillation.
[9] A use of an anti-human NGF antibody or an antigen binding fragment thereof in the manufacture of a pharmaceutical composition for suppressing atrial fibrillation.
[10] A use of an anti-human NGF antibody or an antigen binding fragment thereof for suppressing atrial fibrillation.
[11] An anti-human NGF antibody or an antigen binding fragment thereof for use in suppression of atrial fibrillation.
[12] A method for suppressing atrial fibrillation comprising administering an effective amount of an anti-human NGF antibody or an antigen binding fragment thereof.

### ADVANTAGEOUS EFFECTS OF INVENTION

Since the pharmaceutical composition of the present invention comprising an anti-human NGF antibody or an antigen binding fragment thereof as the active ingredient suppressed the occurrence of atrial fibrillation in a canine model of atrial fibrillation induced by aseptic pericarditis, it is expected to have effect in suppressing atrial fibrillation.

### BRIEF DESCRIPTION OF DRAWINGS

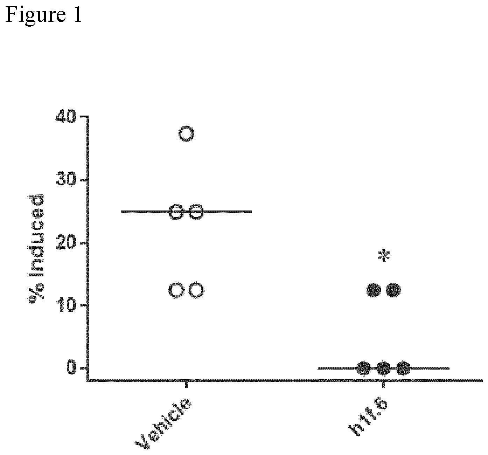

[Figure 1] Fig. 1 is a graph showing the result of Example 1. The y-axis shows the frequency (% Induced) of atrial fibrillation induction when a stimulation using burst pacing (stimulation conditions: stimulation voltage 9.9 V; stimulation interval 90 ms; number of stimulation 20 times; 4 stimulation sites (auricle, low lateral wall, high lateral wall and free wall of the right atrium); number of induction 2 times per stimulation site) was performed on the right atrium in a canine model of atrial fibrillation induced by aseptic pericarditis, and the frequency is shown by the individual value and the median value. "h1f.6" indicates an h1f.6 antibody administered group. * indicates that a significant difference (p<0.05) was recognized against the solvent group (Vehicle) in the Wilcoxon rank sum test.

### DESCRIPTION OF EMBODIMENTS

The present invention is described in detail below, but these descriptions do not limit the scope of the invention. Unless otherwise defined herein, scientific terms and technical terms used in relation to the present invention hold the meaning generally understood by a person skilled in the art.

The present invention relates to a pharmaceutical composition for suppressing atrial fibrillation, having an anti-human NGF antibody or an antigen binding fragment thereof as an active ingredient. The basic structure of an antibody molecule is common among the respective antibody classes and is constituted with heavy-chains having a molecular weight of 50,000 to 70,000 and light-chains having a molecular weight of 20,000 to 30,000. The heavy-chain generally consists of a polypeptide chain including about 440 amino acids, and each class has its characteristic structure. The heavy-chains are called γ, µ, α, δ, and ε chains corresponding to IgG, IgM, IgA, IgD, and IgE. Furthermore, IgG has subclasses such as IgG1, IgG2, IgG3, and IgG4, and these chains are called γ1, γ2, γ3, and γ4 respectively. The light-chain generally consists of a polypeptide chain including about 220 amino acids, and two types of the light-chain including an L-type and a K-type light-chains are known, which are called λ and κ chains respectively. Regarding the peptide constitution of the basic structure of an antibody molecule, two homologous heavy-chains and two homologous light-chains are bound via disulfide bonds (S-S bonds) and non-covalent bonds, and the molecular weight thereof is 150,000 to 190,000. The two kinds of light-chains can be paired with any heavy-chain. Each antibody molecule always consists of two identical light-chains and two identical heavy-chains.

There are four intrachain S-S bonds in a heavy-chain (five bonds for µ and ε chains) and two in a light-chain. One loop is formed per 100 to 110 amino acid residues, and this steric structure is similar among the respective loops and is called a structural unit or a domain. For both heavy-chains and light-chains, the amino acid sequence of the domain positioned at the N-terminal thereof is not constant, even in a reference standard from the same class (subclass) of the same animal species, and this domain is called a variable region. Each of the domains is called a heavy-chain variable region (VH) and a light-chain variable region (VL) respectively. Since the amino acid sequence of the C-terminal side from the variable region is almost constant in each class or subclass, this region is called a constant region, and each of the domains are described as CH1, CH2, CH3 and CL, respectively.

The antigenic determinant site of an antibody is constituted with VH and VL, and the binding specificity depends on the amino acid sequence of this site. On the other hand, biological activities such as binding to complements or various cells reflect the differences in the constant region structure among the various classes of Ig. It is known that the variability in the variable regions of the heavy-chain and light-chain is mostly limited to three small hypervariable regions present in both chains, and these regions are called complementarity determining regions (CDRs; CDR1, CDR2 and CDR3 starting from the N-terminal side). The remaining portion of the variable region is called a framework region (FR) and is relatively constant.

In one aspect, the anti-human NGF antibody or antigen binding fragment thereof that is an active ingredient in the pharmaceutical composition of the present invention, is an antibody or an antigen binding fragment thereof having the feature of (1) or (2) shown below.
(1) An anti-human NGF antibody or an antigen binding fragment thereof comprising: a heavy-chain variable region including CDR1 consisting of an amino acid sequence at positions 31-35 of SEQ ID NO: 3, CDR2 consisting of an amino acid sequence at positions 50-65 of SEQ ID NO: 3, and CDR3 consisting of an amino acid sequence at positions 98-110 of SEQ ID NO: 3; and a light-chain variable region including CDR1 consisting of an amino acid sequence at positions 24-39 of SEQ ID NO: 4, CDR2 consisting of an amino acid sequence at positions 55-61 of SEQ ID NO: 4, and CDR3 consisting of an amino acid sequence at positions 94-102 of SEQ ID NO: 4;
(2) An anti-human NGF antibody or an antigen binding fragment thereof comprising: a heavy-chain variable region consisting of an amino acid sequence at positions 1-121 of SEQ ID NO: 3; and a light-chain variable region consisting of an amino acid sequence at positions 1-113 of SEQ ID NO: 4.

A region between the CH1 domain and the CH2 domain of the heavy-chain constant region of an antibody is called a hinge region. This region includes lots of proline residues and has a plurality of inter-chain S-S bonds connecting two heavy-chains. For example, each hinge region of human IgG1, IgG2, IgG3, and IgG4 includes 2, 4, 11, and 2 cysteine residues respectively which constitute the inter-heavy-chain S-S bonds. The hinge region is a region highly sensitive to a proteolytic enzyme such as papain or pepsin. When an antibody is digested with papain, its heavy-chain is cleaved at a position closer to the N-terminal side than to the inter-heavy-chain S-S bond of the hinge region, whereby the antibody is broken down into two Fab fragments and one Fc fragment. The Fab fragment is constituted with a light-chain and a heavy-chain fragment including a heavy-chain variable region, a CH1 domain, and a portion of the hinge region. When an antibody is digested with pepsin, its heavy-chain is cleaved at a position closer to the C-terminal side than to the inter-heavy-chain S-S bond of the hinge region, whereby F(ab')₂ fragments are generated. The F(ab')₂ fragment is a fragment having a dimeric structure in which two Fab' fragments bind to each other via the inter-heavy-chain S-S bond in the hinge region. The Fab' fragment is constituted with a light-chain and a heavy-chain fragment including a heavy-chain variable region, a CH1 domain, and a portion of the hinge region. Cysteine residues constituting the inter-heavy-chain S-S bond are included in the portion of the hinge region. All of the Fab fragment, F(ab')₂ fragment, and Fab' fragment include the variable region and have antigen-binding activity. In one aspect, the antigen binding fragment of anti-human NGF antibody that is an active ingredient of the pharmaceutical composition of the present invention is a Fab fragment, F(ab')₂ fragment, or Fab' fragment of an anti-human NGF antibody. In another aspect, the antigen binding fragment of the anti-human NGF antibody, which is an active ingredient in the pharmaceutical composition of the present invention, is a Fab fragment of an anti-human NGF antibody. Further, in one aspect, the anti-human NGF antibody or antigen binding fragment thereof that is an active ingredient in the pharmaceutical composition of the present invention, is an antibody or an antigen binding fragment thereof having the feature of (a) and/or (b) shown below:
(a) an anti-human NGF antibody Fab fragment comprising a heavy-chain fragment consisting of an amino acid sequence shown by SEQ ID NO: 3 and a light chain consisting of an amino acid sequence shown by SEQ ID NO: 4;
(b) an anti-human NGF antibody Fab fragment formed by a posttranslational modification of the anti-human NGF antibody Fab fragment of (a).

In one aspect, the anti-human NGF antibody Fab fragment that is an active ingredient in the pharmaceutical composition of the present invention is a Fab fragment with the following features:
An anti-human NGF antibody Fab fragment comprising a heavy-chain fragment consisting of the amino acid sequence shown by SEQ ID NO: 3 and a light-chain consisting of the amino acid sequence shown by SEQ ID NO: 4.

An example of an anti-human NGF antibody Fab fragment that is an active ingredient in the pharmaceutical composition of the present invention is an h1f.6 antibody described in the Examples below.

The anti-human NGF antibody or antigen binding fragment thereof that is an active ingredient in the pharmaceutical composition of the present invention may be readily prepared by a person skilled in the art using methods commonly known in the art.

To give one example, the anti-human NGF antibody Fab fragment that is an active ingredient in the pharmaceutical composition of the present invention may be prepared according to the method described in WO 2016/190263 or variations of such method. For example, the anti-human NGF antibody Fab fragment may be obtained as an anti-human NGF antibody Fab fragment that is stable and retains high neutralizing activity by modifying a fully human anti-human NGF antibody 1-15 (N52D-A)-Fab' fragment (WO 2013/022083; it is also called as "1-15 (N52D-A)-Fab'" in said document), and screening antibodies using various biological activity tests and physical property tests.

To give another example, the anti-human NGF antibody Fab fragment that is an active ingredient in the pharmaceutical composition of the present invention may be readily prepared by a person skilled in the art based on the sequence information disclosed in the present specification using a commonly known method. For example, the anti-human NGF antibody Fab fragment of the present invention may be produced by synthesizing a polynucleotide comprising a base sequence encoding its heavy-chain fragment and a polynucleotide comprising a base sequence encoding its light chain, and connecting the same to suitable expression vectors. Subsequently, the expression vectors are introduced into culture cells. Finally, when the cells are cultured, the person skilled in the art can obtain monoclonal Fab fragments from the culture supernatant. Such method of synthesizing polynucleotide as well as processes including the introduction of the polynucleotide to an expression vector, the introduction of the expression vector to a culture cell, the incubation of the culture cell, and the purification of the Fab fragment may be performed using various methods commonly known in the field of art, and an illustration of such method is described in WO 2016/190263.

As a polynucleotide comprising a base sequence encoding the heavy-chain fragment of the anti-human NGF antibody Fab fragment consisting of the amino acid sequence shown by SEQ ID NO: 3, a polynucleotide comprising a base sequence shown by SEQ ID NO: 1 may be listed as an example. As a polynucleotide comprising a base sequence encoding the light-chain of the anti-human NGF antibody Fab fragment consisting of the amino acid sequence shown by SEQ ID NO: 4, a polynucleotide comprising a base sequence shown by SEQ ID NO: 2 may be listed as an example.

When an antibody is expressed in cells, it is known that the antibody is subjected to posttranslational modifications. Examples of the posttranslational modifications include a cleavage of lysine at C-terminus of a heavy-chain by a carboxypeptidase, a modification of glutamine or glutamic acid at N-terminus of a heavy-chain or a light-chain to pyroglutamic acid by pyroglutamylation, glycosylation, oxidation, deamidation, glycation, and the like. It is known in the art that such posttranslational modifications occur to various antibodies (J. Pharm. Sci., 2008, Vol.97, pp. 2426-2447).

The anti-human NGF antibody Fab fragment that is the active ingredient in the pharmaceutical composition of the present invention may include an anti-human NGF antibody Fab fragment which is subjected to the posttranslational modification(s). An example of the anti-human NGF antibody Fab fragment which may be subjected to posttranslational modification(s) include, an anti-human NGF antibody Fab fragment of which N-terminus of the heavy-chain variable region is pyroglutamylated. Such posttranslational modification through pyroglutamylation at N-terminus is known in the art as not affecting the activity of an antibody (Anal. Biochem., 2006, Vol.348, pp.24-39).

For example, the anti-human NGF antibody Fab fragment that is an active ingredient in the pharmaceutical composition of the present invention includes the following anti-human NGF antibody Fab fragment:
An anti-human NGF antibody Fab fragment formed by a posttranslational modification of an anti-human NGF antibody Fab fragment comprising a heavy-chain fragment consisting of an amino acid sequence shown by SEQ ID NO: 3 and a light chain consisting of an amino acid sequence shown by SEQ ID NO: 4.

As an another example, the anti-human NGF antibody Fab fragment that is an active ingredient in the pharmaceutical composition of the present invention includes the following anti-human NGF antibody Fab fragment:
An anti-human NGF antibody Fab fragment comprising a heavy-chain fragment consisting of the amino acid sequence shown by SEQ ID NO: 3, in which a glutamine at amino acid position 1 of SEQ ID NO: 3 is modified to a pyroglutamic acid, and a light-chain consisting of the amino acid sequence shown by SEQ ID NO: 4.

As a further example, the anti-human NGF antibody Fab fragment that is an active ingredient in the pharmaceutical composition of the present invention includes the following anti-human NGF antibody Fab fragment:
An anti-human NGF antibody Fab fragment comprising a heavy-chain fragment consisting of an amino acid sequence shown by SEQ ID NO: 3 and a light chain consisting of an amino acid sequence shown by SEQ ID NO: 4, and an anti-human NGF antibody Fab fragment comprising a heavy-chain fragment consisting of an amino acid sequence shown by SEQ ID NO: 3, in which glutamine at amino acid position 1 of SEQ ID NO: 3 is modified to pyroglutamic acid, and a light chain consisting of an amino acid sequence shown by SEQ ID NO: 4.

The anti-human NGF antibody or antigen binding fragment thereof that is an active ingredient in the pharmaceutical composition of the present invention binds to human NGF. In addition, the anti-human NGF antibody or antigen binding fragment thereof that is an active ingredient in the pharmaceutical composition of the present invention has a neutralizing activity against human NGF. Methods for measuring the binding activity or neutralizing activity of the anti-human NGF antibody or antigen binding fragment thereof to the human NGF may be performed using various methods commonly known in the art, of which one example is a method described in WO 2016/190263. Methods for evaluating the various stabilities of the anti-human NGF antibody or antigen binding fragment thereof (e.g. thermostability, prolonged storage stability, and high concentration stability) may also make use of the method described in WO 2016/190263. For example, a method that utilizes measurement of aggregation during storage by size exclusion chromatography may be used.

In order to perform an evaluation of the atrial fibrillation suppression effect exerted by the anti-human NGF antibody or antigen binding fragment thereof, it is also possible to perform an *in vivo* study. As an example, an *in vivo* atrial fibrillation suppression effect exerted by the anti-human NGF antibody or antigen binding fragment thereof may be evaluated by performing a test of atrial fibrillation suppression effect that uses a canine model of atrial fibrillation induced by aseptic pericarditis (J Am Coll Cardiol, 1986, Vol. 8, pp.872-879) known to reflect atrial fibrillation.

The anti-human NGF antibody or antigen binding fragment thereof may be optionally purified, and subsequently formulated by a common method to be used in suppressing atrial fibrillation.

"Atrial fibrillation" is a type of tachyarrhythmia, and cardiac failure and cerebral infarction may be listed as a disease relating to tachyarrhythmia. The "atrial fibrillation" used herein also includes postoperative atrial fibrillation that occurs at a high frequency after thoracic surgeries and the like.

The "atrial fibrillation suppression" used herein means suppressing atrial fibrillation, and more specifically, it is a concept that includes preventing the occurrence of atrial fibrillation by applying the pharmaceutical composition of the present invention before the occurrence of atrial fibrillation, and treating atrial fibrillation by applying the pharmaceutical composition of the present invention after the occurrence of atrial fibrillation. In other words, the pharmaceutical composition for suppressing atrial fibrillation in the present invention may be a pharmaceutical composition for prophylaxis of atrial fibrillation or a pharmaceutical composition for treatment of atrial fibrillation. Also, the pharmaceutical composition of the present invention is expected to be effective in suppressing postoperative atrial fibrillation that occurs frequently after thoracic surgeries, that is, in the prophylaxis or treatment of postoperative atrial fibrillation. Hence, in one aspect, the pharmaceutical composition for suppressing atrial fibrillation of the present invention may be a pharmaceutical composition for suppressing postoperative atrial fibrillation, and in one aspect, the pharmaceutical composition for suppressing postoperative atrial fibrillation of the present invention may be a pharmaceutical composition for prophylaxis of postoperative atrial fibrillation or a pharmaceutical composition for treatment of postoperative atrial fibrillation.

The pharmaceutical composition of the present invention comprises pharmaceutically acceptable carriers or additives. The type of pharmaceutically acceptable carriers or additives is not particularly limited, and carriers or additives well known to a person skilled in the art may be used. As examples of carriers that may be included as the pharmaceutical composition of the present invention, distilled water for injection or physiological saline may be listed, and as examples of additives that may be included in the pharmaceutical composition of the present invention, an isotonizing agent, a buffer, a preservative, a humectant, an emulsifier, a dispersant, a stabilizer or a solubilizer may be listed.

The pharmaceutical composition of the present invention may be sterilized by filter filtration, high pressure steam treatment, dry heat treatment, ethylene oxide gas treatment, ultraviolet irradiation, radiation irradiation or addition of a disinfectant. The pharmaceutical composition of the present invention may also be used by producing a sterile solid composition and dissolving or suspending the composition in sterile water or a sterile solvent for injection prior to use.

As examples of the shape of the formulation of the pharmaceutical composition of the present invention, there may be listed non-oral agents such as injection, drops, and depots. The pharmaceutical composition of the present invention may also take the form of a sterile aqueous or non-aqueous solution, suspension or emulsion. As an aqueous solution, distilled water for injection or physiological saline may be listed. As a non-aqueous solvent, alcohols such as ethanol may be listed.

The administrative form of the pharmaceutical composition of the present invention is not particularly limited, and the pharmaceutical composition may be administered by an injection into the fat pad, an intramuscular injection, a subcutaneous injection, or an intravenous injection to the local target tissue. The dose of the anti-human NGF antibody or antigen binding fragment thereof contained in the pharmaceutical composition of the present invention may vary by the level of the symptom and age of the patient, the shape of the pharmaceutical formulation to be used, the form of administration, or the valence of the antibody, but an amount of about 0.001 mg/kg to 100 mg/kg may be used. Also, it can be formulated by adding the anti-human NGF antibody or antigen binding fragment thereof in the present invention in a corresponding amount to the dose described above.

The present invention also relates to the use of anti-human NGF antibody or antigen binding fragment thereof in the manufacture of a pharmaceutical composition for suppressing atrial fibrillation. The present invention also relates to the use of an anti-human NGF antibody or an antigen binding fragment thereof for suppressing atrial fibrillation. Further, the present invention also relates to the anti-human NGF antibody or an antigen binding fragment thereof for use in suppression of atrial fibrillation. In addition, the present invention also relates to the method for suppressing atrial fibrillation comprising administering to a subject an effective amount of an anti-human NGF antibody or an antigen binding fragment thereof. Note that a "subject" is a human or other mammalians requiring the treatment, and one aspect is a human in need of the treatment. The effective amount of the pharmaceutical composition in the suppression method of the present invention may be the same as the amount of an anti-human NGF antibody or the antigen-binding fragment thereof contained in said pharmaceutical composition. The pharmaceutical composition of the present invention may be a pharmaceutical composition administered or used on a site requiring treatment, or sites neighboring that site, particularly the target tissue such as the tissue dissected in a surgical operation or the surrounding area. For example, the pharmaceutical composition may be administered by injection into a fat pad, intramuscular injection or subcutaneous injections to the local target tissue as mentioned above. The present invention includes a case of the anti-human NGF antibody or an antigen-binding fragment thereof being retained at the local target tissue for a certain time, preferably 168 h., and more preferably 96 h.

Although the present invention has been generally described above, specific examples are provided herein only for a better understanding of the present invention. These examples are for illustrative purposes only and do not limit the scope of the present invention.

### EXAMPLES

In steps using a commercially available kit or reagent, experiments were performed according to the attached protocols unless otherwise specified.

An anti-human NGF antibody Fab fragment h1f.6 (also referred to as h1f.6 antibody) that was prepared by the method described in Example 1 of WO 2016/190263 was used. The base sequence of the heavy-chain fragment of hlf.6 antibody is shown as SEQ ID NO: 1, and the amino acid sequence encoded thereby, as SEQ ID NO: 3. The base sequence of the light chain of h1f.6 antibody is shown as SEQ ID NO: 2, and the amino acid sequence encoded thereby, as SEQ ID NO: 4. Note that in Example 1 of WO 2016/190263, it is indicated that a peak which is considered as pyroglutamylation at the N-terminal was confirmed for the majority of the antibody as a result of performing mass spectrometry to analyze the posttranslational modification of a purified h1f.6 antibody.

### <Example 1: Evaluation of the atrial fibrillation suppression effect in a canine model of atrial fibrillation induced by aseptic pericarditis>

The atrial fibrillation suppression effect of h1f.6 antibody in a canine model of atrial fibrillation induced by aseptic pericarditis (J Am Coll Cardiol, 1986, Vol.8, pp.872-879) known to reflect atrial fibrillation was evaluated.

Specifically, solvent (20 mM sodium citrate, 210 mM sucrose, 0.02% polysorbate 80 containing aqueous solution (pH 6)) groups and h1f.6 antibody (administration amount per dog 125 mg, antibody concentration in analyte for administration 125 mg/mL) groups were assigned with each group consisting of 5 dogs. Male beagle dogs (Marshall BioResources Japan) were induced anesthesia by intravenously using thiopental (Mitsubishi Tanabe Pharma Co., Ltd., Labnal) 1 mg/kg, and thereafter tracheal intubation was performed promptly, and anesthesia during surgery was maintained using isoflurane (Mylan Pharmaceutical Co., Ltd., isoflurane inhalation anesthetic solution "Pfizer", 1.4 to 3.0% anesthetic concentration) and oxygen (ventilation frequency: 10 to 15 times/minute, ventilation volume: 11 to 20 mL/kg, end-tidal carbon dioxide tension: 30 to 38 mmHg). After performing thoracotomy at the fifth right intercostal space, then the pericardial membrane was dissected, and the analyte for administration were injected at 1.25 mL each to the fat pad neighboring the anterior right ganglionated plexi (ARGP) and the inferior right ganglionated plexi (IRGP). Subsequently, physiological saline was added at a proportion of 1.5 mL against 1 g of talc (Maruichi Pharmaceutical Co., Ltd., talc bulk powder "Maruishi") to form a mixture, and about 2 g of the mixture was deposited on gauze of 3 ×6 cm (for the right atrium, 1 sheet) and of 3×3 cm (for the left atrium, 2 sheets). Then, the gauze for the right atrium was left on the heart, and the pericardial membrane was sutured to close the chest. Next, thoracotomy was performed at the fifth left intercostal space, after which the pericardial membrane was dissected, and the analyte for administration was injected at 1.25 mL each into the fat pad neighboring the superior left ganglionated plexi (SLGP) and the inferior left ganglionated plexi (ILGP). Then, the gauze for the left atrium was left on the heart for 5 hours, after which the gauzes for both the right/left atriums were taken out. The interior of the pericardium was washed with physiological saline, then the pericardial membrane was sutured to close the chest. Four days after the surgery, an electrode for measuring body surface electrocardiogram was attached under anesthesia, and electrodes (J-shaped lead (Boston Scientific, Finline II sterox) and EPS cartels (6Fr)) were inserted into the right auricle and the right atrium, then burst pacing (Nihon Koden Corporation, Cardiac stimulator) was performed while recording the electrocardiogram at the time of induction by using a polygraph (Nihon Koden Corporation) and PowerLab (ADInstruments, ML870). The stimulation condition was stimulation voltage 9.9 V; stimulation interval 90 ms; number of stimulation 20 times; 4 stimulation sites (auricle of the right atrium, low lateral wall, high lateral wall and free wall of the right atrium) and a number of induction 2 times per stimulation site.

The frequency was measured of atrial fibrillation induced as a result of performing stimulation control on each dog. The median value of the atrial fibrillation induction rate (% Induced) in the solvent group and the h1f.6 antibody group were 25% and 0%, respectively (The arithmetic means thereof were 22.5% and 5.0%,respectively), and the h1f.6 antibody group exhibited a significant reduction (p<0.05) against the solvent group in the Wilcoxon rank sum test (Fig. 1). Based on this result, it was shown that the h1f.6 antibody has a suppression effect against atrial fibrillation.

Based on the above descriptions, it can be seen that the pharmaceutical composition of the present invention comprising an anti-human NGF antibody Fab fragment as an active ingredient may be used as a drug that is useful for suppressing atrial fibrillation.

### INDUSTRIAL APPLICABILITY

The pharmaceutical composition of the present invention comprising an anti-human NGF antibody or antigen binding fragment thereof as an active ingredient is expected to be useful in suppressing atrial fibrillation.

### FREE TEXT OF SEQUENCE LISTING

Explanations are provided for "Artificial Sequence" described for each numerical index <223> of the sequence listing shown below. Specifically, the base sequence shown by SEQ ID NO: 1 of the sequence listing is the base sequence of the heavy-chain fragment of h1f.6 antibody, and the amino acid sequence shown by SEQ ID NO: 3 of the sequence listing is the amino acid sequence of the heavy-chain fragment encoded by SEQ ID NO: 1. The base sequence shown by SEQ ID NO: 2 of the sequence listing is the base sequence of the light chain of hlf.6 antibody, and the amino acid sequence shown by SEQ ID NO: 4 of the sequence listing is the amino acid sequence of the light chain encoded by SEQ ID NO: 2.

## Claims

1. A pharmaceutical composition for suppressing atrial fibrillation comprising an anti-human NGF antibody or an antigen binding fragment thereof, and a pharmaceutically acceptable carrier.

2. The pharmaceutical composition according to claim 1, wherein the antigen binding fragment of the anti-human NGF antibody is an anti-human NGF antibody Fab fragment.

3. The pharmaceutical composition according to claim 2, wherein the anti-human NGF antibody Fab fragment is selected from a group consisting of (a) and/or (b) below:
(a) an anti-human NGF antibody Fab fragment comprising a heavy-chain fragment consisting of an amino acid sequence shown by SEQ ID NO: 3 and a light chain consisting of an amino acid sequence shown by SEQ ID NO: 4;
(b) an anti-human NGF antibody Fab fragment formed by a posttranslational modification of the anti-human NGF antibody Fab fragment of (a).

4. The pharmaceutical composition according to claim 3 comprising an anti-human NGF antibody Fab fragment comprising a heavy-chain fragment consisting of an amino acid sequence shown by SEQ ID NO: 3 and a light chain consisting of an amino acid sequence shown by SEQ ID NO: 4.

5. The pharmaceutical composition according to claim 3 comprising an anti-human NGF antibody Fab fragment formed by a posttranslational modification of an anti-human NGF antibody Fab fragment comprising a heavy-chain fragment consisting of an amino acid sequence shown by SEQ ID NO: 3 and a light chain consisting of an amino acid sequence shown by SEQ ID NO: 4.

6. The pharmaceutical composition according to claim 5 comprising an anti-human NGF antibody Fab fragment comprising a heavy-chain fragment consisting of an amino acid sequence shown by SEQ ID NO: 3, in which glutamine at amino acid position 1 of SEQ ID NO: 3 is modified to pyroglutamic acid, and a light chain consisting of an amino acid sequence shown by SEQ ID NO: 4.

7. The pharmaceutical composition according to claim 3 comprising an anti-human NGF antibody Fab fragment comprising a heavy-chain fragment consisting of an amino acid sequence shown by SEQ ID NO: 3 and a light chain consisting of an amino acid sequence shown by SEQ ID NO: 4, and an anti-human NGF antibody Fab fragment comprising a heavy-chain fragment consisting of an amino acid sequence shown by SEQ ID NO: 3, in which glutamine at amino acid position 1 of SEQ ID NO: 3 is modified to pyroglutamic acid, and a light chain consisting of an amino acid sequence shown by . SEQ ID NO: 4.

8. The pharmaceutical composition according to any one of claims 1 to 7, wherein the atrial fibrillation is postoperative atrial fibrillation.

9. A use of an anti-human NGF antibody or an antigen binding fragment thereof in the manufacture of a pharmaceutical composition for suppressing atrial fibrillation.

10. A use of an anti-human NGF antibody or an antigen binding fragment thereof for suppressing atrial fibrillation.

11. An anti-human NGF antibody or an antigen binding fragment thereof for use in suppression of atrial fibrillation.

12. A method for suppressing atrial fibrillation comprising administering an effective amount of an anti-human NGF antibody or an antigen binding fragment thereof.
